# EUROPEAN PATENT APPLICATION

(11) **EP 2 992 968 A1**
(43) Date of publication of application: **09.03.2016**
(21) Application number: 15179564.8
(22) Date of filing: 03.08.2015
(51) Int. Cl.: B05B 12/08, B05B 17/06

(54) **NEBULIZER AND CONTROLLING METHOD THEREOF**

(30) Priority: 05.09.2014 TW 103130714
(71) Applicant: Delta Electronics, Inc., Taoyuan Hsien 333 (TW)
(72) Inventor: MA, I-Chen, 320 Taoyuan Hsien (TW); LIN, Jung-Yu, 320 Taoyuan Hsien (TW); SUN, Yang-Sheng, 320 Taoyuan Hsien (TW); PAI, Sheng-Wen, 320 Taoyuan Hsien (TW)
(74) Representative: 2K Patentanwälte Blasberg Kewitz & Reichel

(57) **Abstract**

A nebulizer (1) includes a nebulizing circuit (10), a power circuit (11), a sampling circuit (12), a filtering circuit (13) and a controlling circuit (14). The nebulizing circuit (10) includes a piezoelectric actuator (100). During operation of the piezoelectric actuator (100), the liquid is nebulized. The power circuit (11) selectively provides electric power to the nebulizing circuit (10) so as to power the nebulizing circuit (10). A harmonic signal generated by the piezoelectric actuator (100) at a working frequency is sampled by the sampling circuit (12). A specified frequency of the harmonic signal is retrieved by the filtering circuit (13). The controlling circuit (14) controls operations of the power circuit (11) and receiving the specified frequency of the harmonic signal. By comparing the specified frequency with a predetermined frequency correpsonding to the absence of teh liquid, the controlling circuit (14) judges whether the liquid is exhausted. Once the liquid is exhausted, the controlling circuit (14) controls the power circuit (11) to stop providing the electric power to the nebulizing circuit (10).

## Description

### FIELD OF THE INVENTION

The present invention relates to a nebulizer and a controlling method of the nebulizer, and more particularly to a nebulizer and a controlling method of the nebulizer for precisely judging whether the liquid of the nebulizer is exhausted.

### BACKGROUND OF THE INVENTION

The applications of nebulizers are very extensive. For example, the nebulizers may be used to spray environments, moisturize skin or administer medicament. Generally, the nebulizer may be used to nebulize the liquid into mist droplets. Under this circumstance, since the contact area between the liquid and the contacted object is increased, the liquid is easily moisturized or easily absorbed.

Conventionally, the nebulizer uses pressurize gas to nebulize the liquid into mist droplets. With increasing development of science and technology, the nebulizer is developed in view of miniaturization and power-saving efficacy so as to increase the operating efficiency and reduce the occupied space. Consequently, a nebulizer with a piezoelectric actuator made of piezoelectric material has been introduced into the market. In the piezoelectric type nebulizer, the piezoelectric actuator vibrates to nebulize the liquid into mist droplets. Since the piezoelectric type nebulizer has the small-sized and power-saving efficacy, the piezoelectric type nebulizer is gradually adopted in many applications.

However, since the conventional piezoelectric type nebulizer has no mechanism for detecting whether the inner liquid is exhausted, some problems may occur. For example, if the inner liquid is exhausted during operation of the conventional piezoelectric type nebulizer, the conventional piezoelectric type nebulizer is still operated. That is, even if the inner liquid is exhausted, the piezoelectric type nebulizer is not disabled. Since the conventional piezoelectric type nebulizer continuously consume electricity, the problem of wasting electric energy occurs. For solving these problems, a piezoelectric type nebulizer with an automatic power-off function is disclosed. If a detecting unit detects that the inner liquid is exhausted, the piezoelectric type nebulizer is automatically powered off. However, the detecting result of the detecting unit is easily interfered by some factors. For example, the noise generated by the electronic components of the nebulizer may disturb the detecting result. If the detecting unit fails to accurately detect the exhausted status of the inner liquid, the automatic power-off function is not accurately implemented. In other words, the power-saving efficacy of the piezoelectric is impaired.

Therefore, there is a need of providing a nebulizer and a controlling method thereof in order to overcome the above drawbacks.

### SUMMARY OF THE INVENTION

The above problems are solved by a nebulizer according to claim 1 and by a controlling method of the nebulizer according to claims 8 and 12. Further advantageous embodiments are the subject-matter of the dependent claims.

The present invention provides a nebulizer and a controlling method thereof. By means of a filtering circuit, a specified frequency a harmonic signal generated by a piezoelectric actuator is retrieved. According to the specified frequency, the controlling circuit can precisely judge whether the liquid of the nebulizer is exhausted. Once the liquid is exhausted, the power circuit stops providing electric power to the nebulizing circuit. Consequently, the power consumption of the nebulizer is reduced.

In accordance with an aspect of the present invention, there is provided a nebulizer for nebulizing a liquid. The nebulizer includes a nebulizing circuit, a power circuit, a sampling circuit, a filtering circuit and a controlling circuit. The nebulizing circuit includes a piezoelectric actuator. During operation of the piezoelectric actuator, the liquid is nebulized. The power circuit is electrically connected with the nebulizing circuit for selectively providing electric power to the nebulizing circuit so as to power the nebulizing circuit. The sampling circuit is electrically connected with the nebulizing circuit. A harmonic signal generated by the piezoelectric actuator at a working frequency is sampled by the sampling circuit. The filtering circuit is electrically connected with the sampling circuit. A specified frequency of the harmonic signal is retrieved by the filtering circuit. The controlling circuit is electrically connected with the power circuit and the filtering circuit for controlling operations of the power circuit and receiving the specified frequency of the harmonic signal. By comparing the specified frequency with a predetermined frequency, the controlling circuit judges whether the liquid is exhausted. Once the liquid is exhausted, the controlling circuit controls the power circuit to stop providing the electric power to the nebulizing circuit.

In accordance with another aspect of the present invention, there is provided a controlling method of a nebulizer. The nebulizer includes a nebulizing circuit and a power circuit. The nebulizing circuit includes a piezoelectric actuator for nebulizing a liquid. The power circuit selectively provides electric power to the nebulizing circuit. In a step (a), the nebulizer is turned on, so that the power circuit provides the electric power to the nebulizing circuit. In a step (b), a specified frequency of a harmonic signal generated by the piezoelectric actuator at a working frequency is retrieved. A step (c) is performed to judge whether the liquid is exhausted by comparing the specified frequency with a predetermined frequency. In a step (d), if a result of the step (c) indicates that the liquid is exhausted, the electric power is not provided to the nebulizing circuit.

In accordance with a further aspect of the present invention, there is provided a controlling method of a nebulizer. The nebulizer includes a nebulizing circuit and a power circuit. The nebulizing circuit includes a piezoelectric actuator for nebulizing a liquid. The power circuit selectively provides electric power to the nebulizing circuit. In a step (a), the nebulizer is turned on, so that the power circuit provides the electric power to the nebulizing circuit. In a step (b), a specified frequency of a harmonic signal generated by the piezoelectric actuator at a working frequency is retrieved. A step (c) is performed to judge whether the liquid is exhausted by comparing the specified frequency with a predetermined frequency. In a step (d), if the result of the step (c) indicates that the liquid is exhausted, a counting value is added by 1. A step (e) is performed to judge whether the counting value is equal to a predetermined value. In a step (f), if a result of the step (e) indicates that the counting value is equal to the predetermined value, the electric power is provided to the nebulizing circuit.

The above contents of the present invention will become more readily apparent to those ordinarily skilled in the art after reviewing the following detailed description and accompanying drawings, in which:

### BRIEF DESCRIPTION OF THE DRAWINGS

FIG. 1 is a schematic circuit block diagram of a nebulizer according to an embodiment of the present invention;
FIG. 2 is a flowchart illustrating a controlling method of a nebulizer according to a first embodiment of the present invention; and
FIG. 3 is a flowchart illustrating a controlling method of a nebulizer according to a second embodiment of the present invention.

### DETAILED DESCRIPTION OF THE PREFERRED EMBODIMENT

The present invention will now be described more specifically with reference to the following embodiments. It is to be noted that the following descriptions of preferred embodiments of this invention are presented herein for purpose of illustration and description only. It is not intended to be exhaustive or to be limited to the precise form disclosed.

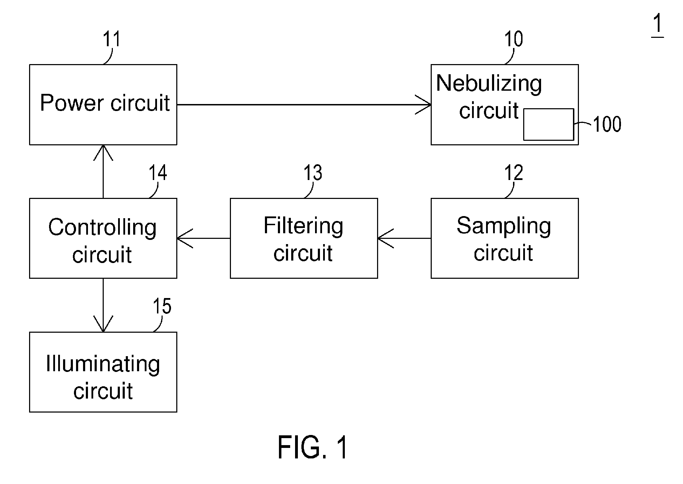

FIG. 1 is a schematic circuit block diagram of a nebulizer according to an embodiment of the present invention. The nebulizer 1 is used to nebulize the liquid into mist droplets. As shown in FIG. 1, the nebulizer 1 comprises a nebulizing circuit 10, a power circuit 11, a sampling circuit 12, a filtering circuit 13 and a controlling circuit 14. By the nebulizing circuit 10, the liquid stored in a reservoir (not shown) is nebulized into mist droplets. The nebulizing circuit 10 comprises a piezoelectric actuator 100. During operation of the nebulizing circuit 10, the piezoelectric actuator 100 vibrates at a specified working frequency. Consequently, the liquid in contact with the piezoelectric actuator 100 is ejected out through a nozzle (not shown) of the nebulizer 1.

In this embodiment, the piezoelectric actuator 100 has composite impedance properties of a capacitor, a resistor and an inductor. Consequently, when the piezoelectric actuator 100 is operated at the working frequency, a harmonic signal is generated because of a resonant effect of the capacitor and the inductor. There is a frequency shift between the frequency of the harmonic signal corresponding to the presence of the liquid and the frequency of the harmonic signal corresponding to the absence of the liquid. Moreover, the working frequency of the piezoelectric actuator 100 may be fixed or varied.

The power circuit 11 is electrically connected with the nebulizing circuit 10 for selectively providing electric power to the nebulizing circuit 10 in order to power the nebulizing circuit 10 and the piezoelectric actuator 100. In an embodiment, the power circuit 11 may receive utility power and convert the utility power into the electric power. In some other embodiments, the power circuit 11 may receive storage power from a battery and convert the storage power into the electric power.

The sampling circuit 12 is electrically connected with the nebulizing circuit 10. The harmonic signal generated by the piezoelectric actuator 100 at the working frequency is sampled by the sampling circuit 12. The filtering circuit 13 is electrically connected with the sampling circuit 12. A specified frequency of the harmonic signal is retrieved by the filtering circuit 13.

The controlling circuit 14 is electrically connected with the power circuit 11 and the filtering circuit 13 for controlling operations of the power circuit 11. Moreover, the specified frequency of the harmonic signal is received by the controlling circuit 14. By comparing the specified frequency with a predetermined frequency stored in the controlling circuit 14, the controlling circuit 14 may judge whether the liquid is exhausted. Once the liquid is exhausted, the power circuit 11 stops providing electric power to the nebulizing circuit 10. Consequently, the power consumption of the nebulizer 1 is reduced.

As mentioned above, there is a frequency shift between the frequency of the harmonic signal corresponding to the presence of the liquid and the frequency of the harmonic signal corresponding to the absence of the liquid. In this embodiment, the frequency of the harmonic signal corresponding to the absence of the liquid may be set as the predetermined frequency of the controlling circuit 14. Consequently, by comparing the specified frequency with the predetermined frequency, the controlling circuit 14 may judge whether the liquid is exhausted and perform a corresponding controlling action. Moreover, the harmonic signal generated by the piezoelectric actuator 100 not only contains the signal component indicating whether the liquid is exhausted but also contains other signal components about the operations of other electronic components of the nebulizer 1. Consequently, by means of the filtering circuit 13, the specified frequency of the signal component of the harmonic signal generated by the piezoelectric actuator 100 to indicate whether the liquid is exhausted will be retrieved. Under this circumstance, the nebulizer 1 can precisely judge whether the liquid is exhausted. Once the liquid is exhausted, the power circuit 11 stops providing electric power to the nebulizing circuit 10. Consequently, the power consumption of the nebulizer 1 is reduced.

In some embodiments, the type of the filtering circuit 13 may be determined according to the range of the specified frequency to be retrieved. For example, the filtering circuit 13 is a high-pass filtering circuit, a band-pass filtering circuit or a low-pass filtering circuit. Moreover, if the specified frequency of the harmonic signal generated by the piezoelectric actuator 100 is higher, the signal component of the harmonic signal corresponding to the specified frequency can indicate whether the liquid is exhausted more precisely. Consequently, it is preferred that the filtering circuit 13 is a high-pass filtering circuit. Under this circumstance, the specified frequency of the harmonic signal retrieved by the filtering circuit 13 is the higher-order harmonic frequency (e.g. 100kHz∼MHz).

Please refer to FIG. 1 again. In this embodiment, the nebulizer 1 further comprises an illuminating circuit 15. The illuminating circuit 15 is in communication with the controlling circuit 14. An example of the illuminating circuit 15 includes but is not limited to a light emitting diode. If the liquid of the nebulizer 1 is not exhausted, the controlling circuit 14 controls the power circuit 11 to provide electric power to the nebulizing circuit 10. Moreover, if the liquid of the nebulizer 1 is not exhausted, the controlling circuit 14 controls the illuminating circuit 15 to continuously emit a light beam so as to notify the user that the nebulizer 1 still contains the liquid. If the controlling circuit 14 judges that the liquid is exhausted by comparing the specified frequency with the predetermined frequency, the controlling circuit 14 controls the power circuit 11 to stop providing electric power to the nebulizing circuit 10. Moreover, if the controlling circuit 14 judges that the liquid is exhausted, the light beam emitted by the illuminating circuit 15 is switched from continuous illumination to intermittent illumination under control of the controlling circuit 14. The intermittent illumination of the light beam may notify the user that the liquid of the nebulizer 1 is exhausted. Under this circumstance, the user may replenish the nebulizer 1 with the liquid.

In some situations, the controlling circuit 14 may erroneously judge that the liquid of the nebulizer 1 is exhausted. For example, if the nebulizer 1 is accidently shaken, the liquid within the nebulizer 1 flows along multiple directions. Under this circumstance, the frequency of the harmonic signal generated by the piezoelectric actuator 100 is temporarily changed. Once the specified frequency is equal to the predetermined frequency in a short time, the controlling circuit 14 may erroneously judge that the liquid of the nebulizer 1 is exhausted and thus the controlling circuit 14 may control the power circuit 11 to stop providing the electric power to the nebulizing circuit 10. For avoiding misjudgment, the controlling method of the present invention may be further modified. For example, in another embodiment, the controlling circuit 14 further stores a counting value. When the controlling circuit 14 judges that the liquid is exhausted by comparing the specified frequency with the predetermined frequency, the counting value is added by 1. Then, the controlling circuit 14 judges whether the counting value is equal to a predetermined value. If the counting value is not equal to a predetermined value, the controlling circuit 14 still controls the power circuit 11 to provide the electric power to the nebulizing circuit 10. Once the counting value is equal to a predetermined value, it means that the controlling circuit 14 has judges the absence of the liquid many times. Under this circumstance, the controlling circuit 14 may control the power circuit 11 to stop providing the electric power to the nebulizing circuit 10. Moreover, once the counting value is equal to a predetermined value, the counting value is zeroed by the controlling circuit 14.

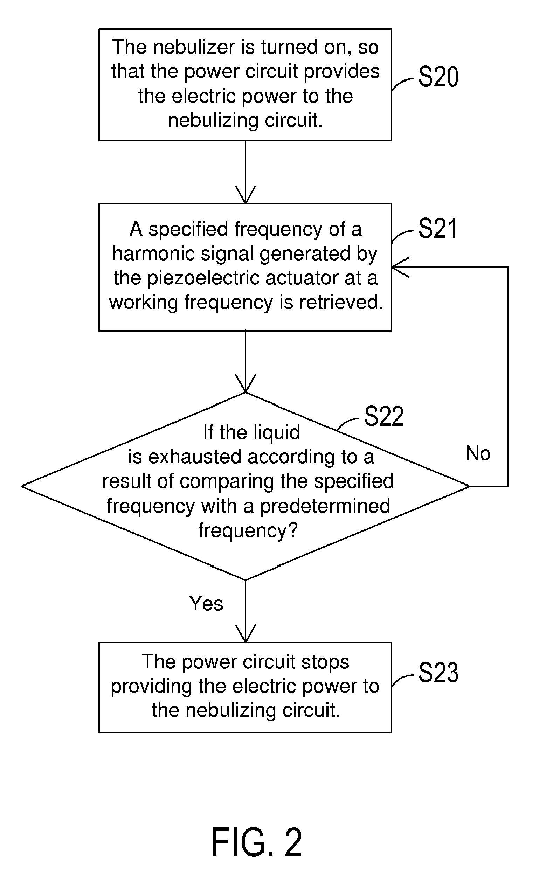

FIG. 2 is a flowchart illustrating a controlling method of a nebulizer according to a first embodiment of the present invention. Please refer to FIGS. 1 and 2. Firstly, in the step S20, the nebulizer 1 is turned on, so that the power circuit 14 provides the electric power to the nebulizing circuit 10. Then, in the step S21, a specified frequency of a harmonic signal generated by the piezoelectric actuator 100 at a working frequency is retrieved. In this step, the harmonic signal generated by the piezoelectric actuator 100 at the working frequency is sampled by the sampling circuit 12, and then the specified frequency of the harmonic signal is retrieved by the filtering circuit 13. Then, in the step S22, the controlling circuit 14 judges whether the liquid is exhausted by comparing the specified frequency with a predetermined frequency. If the judging result of the step S22 indicates that the liquid is exhausted, the step S23 is performed. Under this circumstance, the controlling circuit 14 controls the power circuit 11 to stop providing the electric power to the nebulizing circuit 10. Whereas, if the judging result of the step S22 indicates that the liquid is not exhausted, the step S21 is repeatedly done.

In some embodiments, while nebulizer 1 is turned on and the power circuit 14 provides the electric power to the nebulizing circuit 10 (i.e. in the step S20), the controlling circuit 14 also controls the illuminating circuit 15 to continuously emit a light beam. Moreover, while the controlling circuit 14 controls the power circuit 11 to stop providing the electric power to the nebulizing circuit 10 (i.e. in the step S23), the controlling circuit 14 controls the illuminating circuit 15 to intermittently emit the light beam. The intermittent illumination of the light beam may notify the user that the liquid of the nebulizer 1 is exhausted. Under this circumstance, the user may replenish the nebulizer 1 with the liquid.

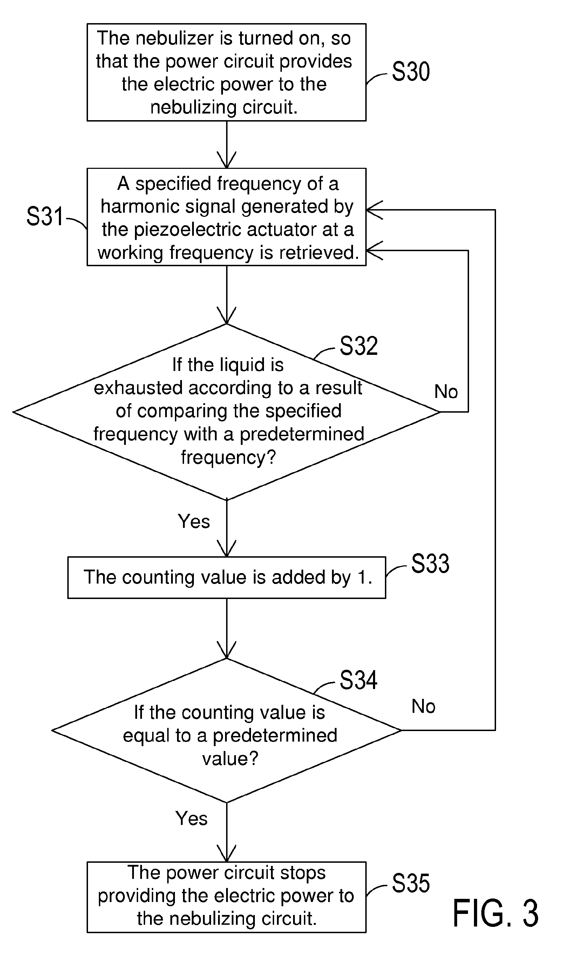

FIG. 3 is a flowchart illustrating a controlling method of a nebulizer according to a second embodiment of the present invention. Please refer to FIGS. 1 and 3. Firstly, in the step S30, the nebulizer 1 is turned on, so that the power circuit 14 provides the electric power to the nebulizing circuit 10. Then, in the step S31, a specified frequency of a harmonic signal generated by the piezoelectric actuator 100 at a working frequency is retrieved. In this step, the harmonic signal generated by the piezoelectric actuator 100 at the working frequency is sampled by the sampling circuit 12, and then the specified frequency of the harmonic signal is retrieved by the filtering circuit 13. Then, in the step S32, the controlling circuit 14 judges whether the liquid is exhausted by comparing the specified frequency with a predetermined frequency. If the judging result of the step S32 indicates that the liquid is exhausted, the step S33 is performed. In the step S33, the counting value stored in the controlling circuit 14 is added by 1. Then, in the step S34, the controlling circuit 14 judges whether the counting value is equal to a predetermined value. If the judging result of the step S34 indicates that the counting value is equal to the predetermined value, the step S35 is performed. Under this circumstance, the controlling circuit 14 controls the power circuit 11 to stop providing the electric power to the nebulizing circuit 10. In addition, the counting value is zeroed by the controlling circuit 14. Whereas, if the judging result of the step S34 indicates that the counting value is not equal to the predetermined value, the step S31 is repeatedly done. Moreover, if the judging result of the step S32 indicates that the liquid is not exhausted, the step S21 is repeatedly done.

In some embodiments, while nebulizer 1 is turned on and the power circuit 14 provides the electric power to the nebulizing circuit 10 (i.e. in the step S30), the controlling circuit 14 also controls the illuminating circuit 15 to continuously emit a light beam. Moreover, while the controlling circuit 14 controls the power circuit 11 to stop providing the electric power to the nebulizing circuit 10 (i.e. in the step S35), the controlling circuit 14 controls the illuminating circuit 15 to intermittently emit the light beam. The intermittent illumination of the light beam may notify the user that the liquid of the nebulizer 1 is exhausted. Under this circumstance, the user may replenish the nebulizer 1 with the liquid.

From the above descriptions, the present invention provides a nebulizer and a controlling method thereof. The nebulizer comprises a nebulizing circuit, a power circuit, a sampling circuit, a filtering circuit and a controlling circuit. By means of the filtering circuit, the specified frequency the harmonic signal generated by the piezoelectric actuator is retrieved. According to the specified frequency, the controlling circuit can precisely judge whether the liquid of the nebulizer is exhausted. Once the liquid is exhausted, the power circuit stops providing electric power to the nebulizing circuit. Consequently, the power consumption of the nebulizer is reduced. Moreover, the controlling circuit further stores a counting value. When the controlling circuit judges the liquid is exhausted by comparing the specified frequency with the predetermined frequency, the counting value is added by 1. Once the counting value is equal to a predetermined value, the controlling circuit controls the power circuit to stop providing the electric power to the nebulizing circuit. Consequently, the problem caused by misjudgment will be overcome.

## Claims

1. A nebulizer (1) for nebulizing a liquid, the nebulizer (1) comprising:
a nebulizing circuit (10) comprising a piezoelectric actuator (100), wherein during operation of the piezoelectric actuator (100), the liquid is nebulized;
a power circuit (11) electrically connected with the nebulizing circuit (10) for selectively providing electric power to the nebulizing circuit (10) so as to power the nebulizing circuit (10);
a sampling circuit (12) electrically connected with the nebulizing circuit (10), wherein a harmonic signal generated by the piezoelectric actuator (100) at a working frequency is sampled by the sampling circuit (12);
a filtering circuit (13) electrically connected with the sampling circuit (12), wherein a specified frequency of the harmonic signal is retrieved by the filtering circuit (13); and
a controlling circuit (14) electrically connected with the power circuit (11) and the filtering circuit (13) for controlling operations of the power circuit (11) and receiving the specified frequency of the harmonic signal, wherein by comparing the specified frequency with a predetermined frequency, the controlling circuit (14) judges whether the liquid is exhausted, wherein once the liquid is exhausted, the controlling circuit (14) controls the power circuit (11) to stop providing the electric power to the nebulizing circuit (10).

2. The nebulizer according to claim 1, wherein the piezoelectric actuator (100) has composite impedance properties of a capacitor, a resistor and an inductor, wherein the working frequency is fixed.

3. The nebulizer according to any of the preceding claims, wherein the filtering circuit (13) is a high-pass filtering circuit, a band-pass filtering circuit or a low-pass filtering circuit.

4. The nebulizer according to claim 3, wherein the filtering circuit (13) is a high-pass filtering circuit, and the specified frequency of the harmonic signal retrieved by the filtering circuit (13) is in a range between 100kHz and 1MHz.

5. The nebulizer according to any of the preceding claims, wherein the nebulizer (1) further comprises an illuminating circuit (15), and the illuminating circuit (15) is electrically connected with the controlling circuit (14), wherein while the controlling circuit (14) controls the power circuit (11) to provide electric power to the nebulizing circuit (10), the controlling circuit (14) controls the illuminating circuit (15) to continuously emit a light beam, wherein while the controlling circuit (14) controls the power circuit (11) to stop providing the electric power to the nebulizing circuit (10), the controlling circuit (14) controls the illuminating circuit (15) to intermittently emit the light beam.

6. The nebulizer according to any of the preceding claims, wherein a counting value is stored in the controlling circuit (14), wherein when the controlling circuit (14) judges the liquid is exhausted by comparing the specified frequency with the predetermined frequency, the counting value is added by 1, wherein when the counting value is equal to a predetermined value, the controlling circuit (14) controls the power circuit (11) to stop providing the electric power to the nebulizing circuit (10).

7. The nebulizer according to claim 6, wherein when the counting value is equal to a predetermined value, the counting value is zeroed by the controlling circuit (14).

8. A controlling method of a nebulizer (1), the nebulizer (1) comprising a nebulizing circuit (10) and a power circuit (11), the nebulizing circuit (10) comprising a piezoelectric actuator (100) for nebulizing a liquid, the power circuit (11) selectively providing electric power to the nebulizing circuit (10), the controlling method comprising steps of:
(a) turning on the nebulizer (1), so that the power circuit (11) provides the electric power to the nebulizing circuit (10);
(b) retrieving a specified frequency of a harmonic signal, which is generated by the piezoelectric actuator (100) at a working frequency;
(c) judging whether the liquid is exhausted by comparing the specified frequency with a predetermined frequency; and
(d) if a result of the step (c) indicates that the liquid is exhausted, stopping providing the electric power to the nebulizing circuit (10).

9. The controlling method according to claim 8, wherein if the result of the step (c) indicates that the liquid is not exhausted, the step (b) is repeatedly done.

10. The controlling method according to any of the any of the preceding method claims, wherein the nebulizer (1) further comprises a sampling circuit (12) and a filtering circuit (13), wherein the sampling circuit (12) is electrically connected with the nebulizing circuit (10), and the filtering circuit (13) is electrically connected with the sampling circuit (12), wherein in the step (b), the harmonic signal generated by the piezoelectric actuator (100) at the working frequency is sampled by the sampling circuit (12), and then the specified frequency of the harmonic signal is retrieved by the filtering circuit (13).

11. The controlling method according to any of the preceding method claims, wherein in the step (a), an illuminating circuit (15) of the nebulizer (1) continuously emits a light beam, wherein in the step (d), the illuminating circuit (15) intermittently emits the light beam.

12. A controlling method of a nebulizer (1), the nebulizer (1) comprising a nebulizing circuit (10) and a power circuit (11), the nebulizing circuit (10) comprising a piezoelectric actuator (100) for nebulizing a liquid, the power circuit (11) selectively providing electric power to the nebulizing circuit (10), the controlling method comprising steps of:
(a) turning on the nebulizer (1), so that the power circuit (11) provides the electric power to the nebulizing circuit (10);
(b) retrieving a specified frequency of a harmonic signal, which is generated by the piezoelectric actuator (100) at a working frequency;
(c) judging whether the liquid is exhausted by comparing the specified frequency with a predetermined frequency; and
(d) if the result of the step (c) indicates that the liquid is exhausted, adding 1 to a counting value;
(e) judging whether the counting value is equal to a predetermined value; and
(f) if a result of the step (e) indicates that the counting value is equal to the predetermined value, stopping providing the electric power to the nebulizing circuit (10).

13. The controlling method according to claim 12, wherein in the step (f), the counting value is further zeroed, wherein if the result of the step (c) indicates that the liquid is not exhausted, the step (b) is repeatedly done, and wherein if the result of the step (e) indicates that the counting value is not equal to the predetermined value, the step (b) is repeatedly done.

14. The controlling method according to any of the preceding method claims, wherein in the step (a), an illuminating circuit (15) of the nebulizer (1) continuously emits a light beam, wherein in the step (f), the illuminating circuit (15) intermittently emits the light beam.

15. The controlling method according to any of the preceding method claims, wherein the nebulizer (1) further comprises a sampling circuit (12) and a filtering circuit (13), wherein the sampling circuit (12) is electrically connected with the nebulizing circuit (10), and the filtering circuit (13) is electrically connected with the sampling circuit (12), wherein in the step (b), the harmonic signal generated by the piezoelectric actuator (100) at the working frequency is sampled by the sampling circuit (12), and then the specified frequency of the harmonic signal is retrieved by the filtering circuit (13).
